(19) Europäisches Patentamt
European Patent Office
Office européen des brevets

(11) Veröffentlichungsnummer: 0 028 689
B1

(12) # EUROPÄISCHE PATENTSCHRIFT

(45) Veröffentlichungstag der Patentschrift: 01.12.82

(51) Int. Cl.³: A 61 K 31/41 // C07D249/08, C07C49/16, C07C49/167, C07C49/255, C07C143/68

(21) Anmeldenummer: 80105457.8

(22) Anmeldetag: 12.09.80

(54) **Acylierte Triazolyl-gamma-fluorpinakolyl-Derivate enthaltende antimykotische Mittel und ihre Herstellung.**

(30) Priorität: 24.09.79 DE 2938597

(43) Veröffentlichungstag der Anmeldung: 20.05.81 Patentblatt 81/20

(45) Bekanntmachung des Hinweises auf die Patenterteilung: 01.12.82 Patentblatt 82/48

(84) Benannte Vertragsstaaten: AT BE CH DE FR GB IT LI NL SE

(56) Entgegenhaltungen:
EP-A-0 019 130
DE-A-2 811 916

**Die Akte enthält technische Angaben, die nach dem Eingang der Anmeldung eingereicht wurden und die nicht in dieser Patentschrift enthalten sind.**

(73) Patentinhaber: BAYER AG, Zentralbereich Patente, Marken und Lizenzen, D-5090 Leverkusen 1, Bayerwerk (DE)

(72) Erfinder: Krämer, Wolfgang, Dr., Am Eckbusch 39/162, D-5600 Wuppertal 1 (DE)
Erfinder: Büchel, Karl Heinz, Prof. Dr., Haus an der Dachsdelle Dabringhausener-Strasse 42, D-5093 Burscheid (DE)
Erfinder: Stetter, Jörg, Dr., Gellertweg 4, D-5600 Wuppertal 1 (DE)
Erfinder: Plempel, Manfred, Dr., Pahlkestrasse 5, D-5600 Wuppertal 1 (DE)
Erfinder: Haller, Ingo, Dr., Viktoriastrasse 99, D-5600 Wuppertal 1 (DE)

EP 0 028 689 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Acylierte Triazolyl-gamma-fluorpinakolyl-Derivate enthaltende antimykotische Mittel und ihre Herstellung

Die Erfindung betrifft antimykotische Mittel, welche neue acylierte Triazolyl-γ-fluorpinakolyl-Derivate als Wirkstoffe enthalten.

Es ist bereits bekannt geworden, dass acylierte 1-Triazolyl-2-hydroxy-butan-Derivate, wie insbesondere im Phenylteil substituierte 2-Acyloxy-3,3-dimethyl-1-phenoxyl,1-(1,2,4-triazol-1-yl)-butane, gute fungizide Eigenschaften aufweisen (vgl. DE-OS 26 00 799). Ihre antimykotische Wirksamkeit ist jedoch, insbesondere in-vivo gegen Candida, nicht befriedigend.

Es wurde gefunden, dass die neuen acylierten Triazolyl-γ-fluorpinakolyl-Derivate der allgemeinen Formel I

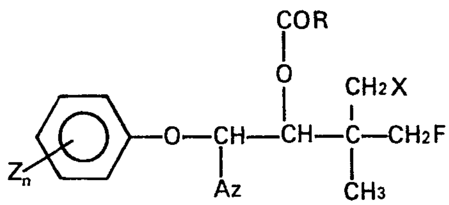 (I)

in welcher

Az für 1,2,4-Triazol-1-yl oder 1,2,4-Triazol-4-yl steht,

R für geradkettiges oder verzweigtes Alkyl mit 1–8 Kohlenstoffatomen; geradkettiges oder verzweigtes Alkenyl und Alkinyl mit jeweils 2–4 Kohlenstoffatomen; Halogenalkyl mit 1–2 Kohlenstoff- und 1–5 Halogenatomen; Alkoxy- und Alkoxyalkyl mit 1–4 Kohlenstoffatomen in jedem Alkylteil; Cycloalkyl mit 5–7 Kohlenstoffatomen; gegebenenfalls durch Halogen, Cyano, Nitro, Alkyl mit 1–2 Kohlenstoffatomen und Alkoxy mit 1–2 Kohlenstoffatomen substituiertes Phenyl, Phenylalkyl und Phenoxyalkyl mit jeweils bis zu 2 Kohlenstoffatomen im Alkylteil; Alkylamino mit 1–12 Kohlenstoffatomen; Dialkylamino mit 1–4 Kohlenstoffatomen in jedem Alkylteil; Halogenalkylamino mit bis zu 4 Kohlenstoff- und bis zu 5 gleichen oder verschiedenen Halogenatomen; Alkoxycarbonylamino und Alkoxyalkylamino mit jeweils 1–4 Kohlenstoffatomen in jedem Alkylteil; gegebenenfalls einfach oder mehrfach durch Halogen, Nitro, Cyano, geradkettiges oder verzweigtes Alkyl mit 1–4 Kohlenstoffatomen, Halogenalkyl mit bis zu 2 Kohlenstoffatomen und bis zu 5 gleichen oder verschiedenen Halogenatomen substituiertes Phenylamino; Alkoxycarbonylalkenyl mit 1–4 Kohlenstoffatomen im Alkylteil und 2–4 Kohlenstoffatomen im Alkenylteil steht;

X für Wasserstoff oder Fluor steht;

Z für Halogen, Cyano, Nitro, geradkettiges oder verzweigtes Alkyl mit bis zu 4 Kohlenstoffatomen; Cycloalkyl mit 5–7 Kohlenstoffatomen; Halogenalkyl mit bis zu 2 Kohlenstoffatomen und bis zu 5 Halogenatomen; Alkoxycarbonyl mit insgesamt bis zu 5 Kohlenstoffatomen; Alkoxy und Alkylthio mit jeweils bis zu 2 Kohlenstoffatomen; gegebenenfalls durch Halogen, Amino, Cyano, Nitro oder Alkyl mit 1–2 Kohlenstoffatomen substituiertes Phenyl und Phenoxy; gegebenenfalls im Alkylteil durch Alkylcarbonyloxy mit insgesamt 3 Kohlenstoffatomen und im Phenylteil durch Halogen, Nitro und Cyano substituiertes Phenylalkyl mit 1–2 Kohlenstoffatomen im Alkylteil steht;

n für ganze Zahlen von 0 bis 3 steht;

und deren physiologisch verträgliche Säureadditions-Salze und Metallkomplexe gute antimykotische Eigenschaften aufweisen.

Die Verbindungen der allgemeinen Formel I besitzen zwei asymmetrische Kohlenstoffatome; sie können deshalb in der erythro- wie in der threo-Form vorliegen. In beiden Fällen liegen sie vorwiegend als Racemate vor.

Überaschenderweise zeigen die neuen acylierten Triazolyl-γ-fluorpinakolyl-Derivate der allgemeinen Formel I eine bessere antimykotische Wirksamkeit, insbesondere in-vivo gegen Candida, als die aus dem Stand der Technik bekannten acylierten 1-Triazolyl-2-hydroxy-butan-Derivate, die chemisch die naheliegendsten Verbindungen sind. Sie stellen somit eine Bereicherung der Pharmazie dar.

Besonders bevorzugt sind diejenigen Verbindungen der allgemeinen Formel I, in denen R für Methyl, Ethyl, Isobutyl, Chlormethyl, Dichlormethyl, Chlorethyl, Chlorpropyl, Methylacryl, Cyclohexyl, gegebenenfalls einfach oder mehrfach substituiertes Phenyl, Benzyl oder Phenoxymethyl mit Chlor, Brom, Methyl oder Methoxy als Substituenten, ferner für Methoxy, Ethoxy, Isopropoxy, Butoxy oder Isobutoxy, Methyl- und Ethylamino, Dimethylamino, Phenylamino, Chlorphenylamino, Chlorethylamino, Methoxycarbonylamino, Ethoxycarbonylamino und Methoxymethylamino steht, X für Wasserstoff oder Fluor steht; Z für Chlor, Brom, Methyl, Ethyl, Cyclohexyl, Methoxy, Methylthio, Trifluormethyl, Methoxycarbonyl, Cyano, Nitro sowie gegebenenfalls durch Chlor substituiertes Phenyl, Benzyl oder Phenoxy steht und n für 0, 1 oder 2 steht.

Im einzelnen seien ausser den Herstellungsbeispielen und den Beispielen der Tabelle 1 folgende Verbindungen der allgemeinen Formel I genannt (Az steht dabei sowohl für 1,2,4-Triazol-1-yl als auch für 1,2,4-Triazol-4-yl):

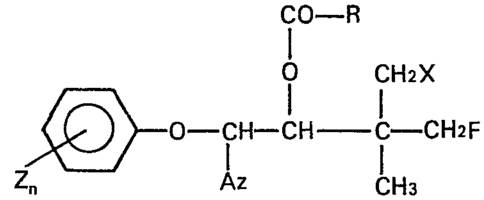 (I)

| R | X | $Z_n$ |
|---|---|---|
| $-CHCl_2$ | H | 4-Cl |
| $-CH_2Cl$ | H | 4-Cl |

| R | X | $Z_n$ |
|---|---|---|
| $-CH_3$ | H | 4– 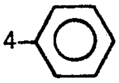 |
| $-NH-$ 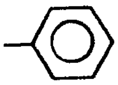 | H | 4– 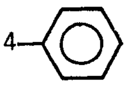 |
| $-NH-$ 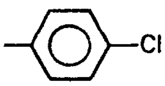 | H | 4– 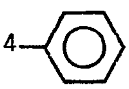 |
| $-NHCH_3$ | H | 4– 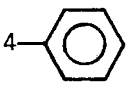 |
| $-NHCH_3$ | H | 4–Cl; 2–$CH_3$ |
| $-NHCH_3$ | H | 2–Cl |
| $-NHC_2H_5$ | H | 4– 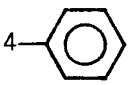 |
| $-C_4H_9$–i | H | 4–Br |
| $-CH_3$ | H | 2,4–$Cl_2$ |
| $-C_2H_5$ | H | 2,4–$Cl_2$ |
| $-CH_3$ | H | 4–$OCH_3$ |
| $-CH_2Cl$ | H | 3–$CF_3$ |
| $-CHCl_2$ | H | 4–CO–$OCH_3$ |
| $-NHCH_2OCH_3$ | H | 4– 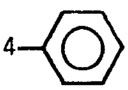 |
| $-NHCH_2OC_2H_5$ | H | 4– 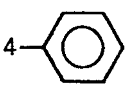 |
| $-NH-COOCH_3$ | H | 4– 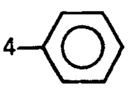 |
| $-NH-COOC_2H_5$ | H | 4– 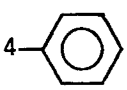 |
| $-N(CH_3)_2$ | H | 4– 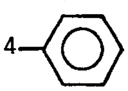 |
| $-OCH_3$ | H | 4– 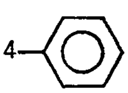 |
| $-CH_3$ | H | 4– 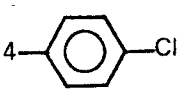 |
| $-CH_3$ | H | 4–O– 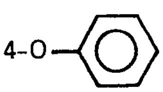 |
| $-CH_3$ | H | 4–O– 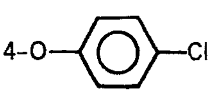 |
| $-CH_3$ | H | 4–CN |
| $-CH_3$ | H | 4–$NO_2$ |
| $-CH_2OC_2H_5$ | H | 2,4–$Cl_2$ |
| $-NHCH(CH_3)_2$ | H | 2,4–$Cl_2$ |
| $-NH-CH_2OCH_3$ | H | 2,4–$Cl_2$ |
| $-OC_2H_5$ | H | 2,4–$Cl_2$ |
| $-C(CH_3)=CH_2$ | H | 2,4–$Cl_2$ |
| $-CH_2-CH(CH_3)_2$ | H | 2,4–$Cl_2$ |

| R | X | $Z_n$ |
|---|---|---|
| $-CH_2-$ 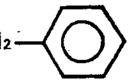 | H | 2,4–$Cl_2$ |
| $-CH_2-CH_2Cl$ | H | 2,4–$Cl_2$ |
| $-CH_2-CH_2-CH_2Cl$ | H | 2,4–$Cl_2$ |
| $-NH-$ 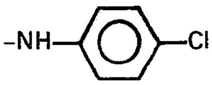 | H | 2,4–$Cl_2$ |
| $-CHCl_2$ | H | 2,4–$Cl_2$ |
| $-CH_2Cl$ | H | 2,4–$Cl_2$ |
| 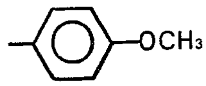 | H | 2,4–$Cl_2$ |
| $-CH_2-O-$ 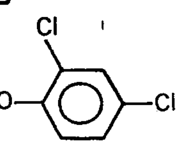 | H | 2,4–$Cl_2$ |
| 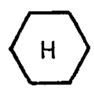 | H | 2,4–$Cl_2$ |
| 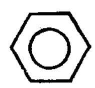 | H | 2,4–$Cl_2$ |
| 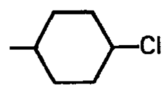 | H | 2,4–$Cl_2$ |
| $-CH_2OC_2H_5$ | H | 4–Cl |
| $-NH-CH(CH_3)_2$ | H | 4–Cl |
| $-NH-CH_2OCH_3$ | H | 4–Cl |
| $-OC_2H_5$ | H | 4–Cl |
| $-C(CH_3)=CH_2$ | H | 4–Cl |
| $-CH_2-CH(CH_3)_2$ | H | 4–Cl |
| $-CH_2-$ 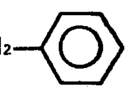 | H | 4–Cl |
| $-CH_2-CH_2Cl$ | H | 4–Cl |
| $-CH_2-CH_2-CH_2Cl$ | H | 4–Cl |
| $-NH-$ 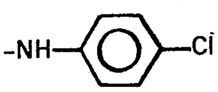 | H | 4–Cl |
| 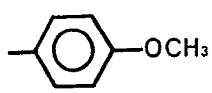 | H | 4–Cl |
| $-CH_2-O-$ 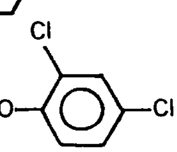 | H | 4–Cl |
| 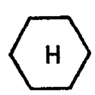 | H | 4–Cl |
| 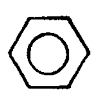 | H | 4–Cl |
| 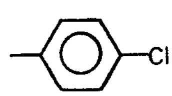 | H | 4–Cl |
| $-NHCH_3$ | F | 2,4–$Cl_2$ |
| $-NHCH_3$ | F | 4–Cl |

| R | X | $Z_n$ |
|---|---|---|
| $-CH_3$ | F | 4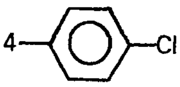 |
| $-NHCH_3$ | F | 4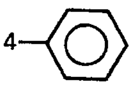 |
| $-NHC_2H_5$ | F | 4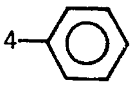 |
| $-NHCH_2OCH_3$ | F | 4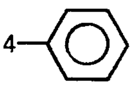 |
| $-NH-CH(CH_3)_2$ | F | 4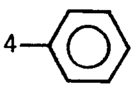 |
| $-CH_2OC_2H_5$ | F | 4-Cl |
| $-NH-CH(CH_3)_2$ | F | 4-Cl |
| $-NHCH_2OCH_3$ | F | 4-Cl |
| $-OC_2H_5$ | F | 4-Cl |
| $-C(CH_3)=CH_2$ | F | 4-Cl |
| $-CH_2-CH(CH_3)_2$ | F | 4-Cl |
| $-CH_2$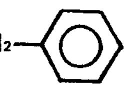 | F | 4-Cl |
| $-CH_2-CH_2Cl$ | F | 4-Cl |
| $-CH_2-CH_2-CH_2Cl$ | F | 4-Cl |
| -NH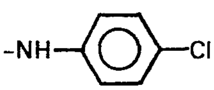 | F | 4-Cl |
| $-CHCl_2$ | F | 4-Cl |
| $-CH_2Cl$ | F | 4-Cl |
| 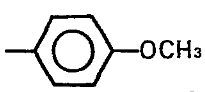 | F | 4-Cl |
| $-CH_2-O$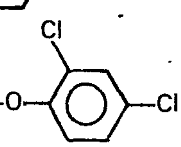 | F | 4-Cl |
| 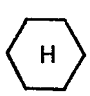 | F | 4-Cl |
| 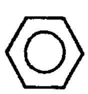 | F | 4-Cl |
| 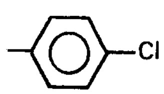 | F | 4-Cl |
| $-CH_2OC_2H_5$ | F | $2,4-Cl_2$ |
| $-NH-CH(CH_3)_2$ | F | $2,4-Cl_2$ |
| $-NH-CH_2OCH_3$ | F | $2,4-Cl_2$ |
| $-OC_2H_5$ | F | $2,4-Cl_2$ |
| $-C(CH_3)=CH_2$ | F | $2,4-Cl_2$ |
| $-CH_2-CH(CH_3)_2$ | F | $2,4-Cl_2$ |
| $-CH_2$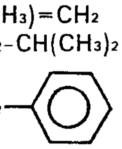 | F | $2,4-Cl_2$ |
| $-CH_2-CH_2Cl$ | F | $2,4-Cl_2$ |
| $-CH_2-CH_2-CH_2Cl$ | F | $2,4-Cl_2$ |
| -NH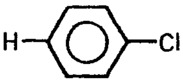 | F | $2,4-Cl_2$ |
| $-CHCl_2$ | F | $2,4-Cl_2$ |
| $-CH_2Cl$ | F | $2,4-Cl_2$ |
| 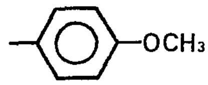 | F | $2,4-Cl_2$ |
| $-CH_2-O$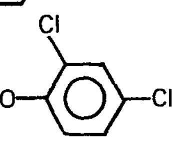 | F | $2,4-Cl_2$ |
| 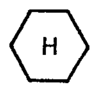 | F | $2,4-Cl_2$ |
| 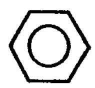 | F | $2,4-Cl_2$ |
| 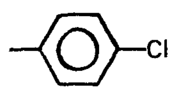 | F | $2,4-Cl_2$ |

Die erfindungsgemässen Wirkstoffe, deren Säureadditionssalze und Metallsalz-Komplexe sind noch nicht bekannt. Sie können jedoch gemäss einem eigenen Vorschlag hergestellt werden, indem man 1-Triazolyl-2-hydroxy-butan-Derivate der allgemeinen Formel II

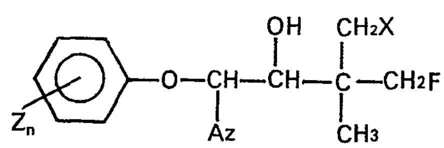
(II)

in welcher
Az, X, Z und n die oben angegebene Bedeutung haben,

a) mit Säurehalogeniden nach bekannten Methoden, z.B. in molaren Mengen in Gegenwart eines inerten organischen Lösungsmittels, z.B. Essigester, bei Temperaturen zwischen 0 und 100°C umsetzt.

Die Verbindungen der allgemeinen Formel I fallen in Form ihrer Hydrohalogenide an und können als solche isoliert werden, indem man sie durch Zugabe eines organischen Solvents, z.B. Hexan, ausfällt, absaugt und gegebenenfalls durch Umkristallisation reinigt. Die Verbindungen der allgemeinen Formel I können auch in Form ihrer freien Basen isoliert werden, indem man das Reaktionsgemisch mit wässriger Natriumhydrogencarbonatlösung versetzt und die Base nach üblichen Methoden isoliert.

Die Verbindungen der allgemeinen Formel I können ferner dadurch hergestellt werden, dass man Verbindungen der allgemeinen Formel II

b) mit Säureanhydriden nach bekannten Methoden, z.B. in molaren Mengen in Gegenwart eines inerten organischen Lösungsmittels, z.B. Aceton oder Überschuss an Säureanhydrid und in Gegenwart eines sauren oder basischen Katalysators, z.B. Natriumacetat, bei Temperaturen zwischen 0 und 150°C umsetzt und die Verbindungen der allgemeinen Formel I in üblicher Weise isoliert, oder

c) mit Ketenen nach bekannten Methoden, z.B. in molaren Mengen in Gegenwart eines inerten organischen Lösungsmittels, z.B. Essigester, und in Gegenwart eines sauren oder basischen Katalysators, z.B. Natriumacetat, bei Temperaturen zwischen -10 und 70°C umsetzt und die Verbindungen der allgemeinen Formel I in üblicher Weise isoliert, oder

d) mit Isocyanaten nach bekannten Methoden, z.B. in molaren Mengen in Gegenwart eines inerten organischen Lösungsmittels, z.B. Essigester, und in Gegenwart eines Katalysators, z.B. Triethylamin, bei Temperaturen zwischen 0 und 100°C umsetzt und die Verbindungen der allgemeinen Formel I in üblicher Weise isoliert und gegebenenfalls durch Umsetzen mit Säuren die Säureadditions-Salze bzw. durch Reaktion mit Metallsalzen die Metallsalz-Komplexe herstellt.

Die 1-Triazolyl-2-hydroxy-butan-Derivate der allgemeinen Formel II sind Gegenstand der DE-OS 2918894 und können hergestellt werden, indem man Halogenetherketone der allgemeinen Formel III

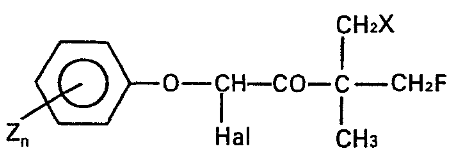

(III)

in welcher
X, Z und n die oben angegebene Bedeutung haben und
Hal für Halogen, vorzugsweise Chlor oder Brom steht,
mit 1,2,4-Triazol in Gegenwart eines Säurebindemittels, wie beispielsweise Natriumcarbonat oder ein Überschuss an Triazol, und gegebenenfalls in Gegenwart eines inerten organischen Lösungsmittels, wie beispielsweise Aceton oder Acetonitril, bei Temperaturen zwischen 60 und 120°C umsetzt und die erhaltenen Keto-Derivate nach bekannten Methoden reduziert, wie z.B. durch Umsetzung mit komplexen Hydriden, wie insbesondere Natriumborhydrid, gegebenenfalls in Gegenwart eines polaren organischen Lösungsmittels, wie beispielsweise Alkohole, bei Temperaturen zwischen 0 und 30°C; oder durch Umsetzung mit Aluminiumisopropylat in Gegenwart eines inerten organischen Lösungsmittels, wie beispielsweise Isopropanol, bei Temperaturen zwischen 20 und 120°C. Die Aufarbeitung erfolgt in üblicher Weise.

Die Halogenetherketone der allgemeinen Formel III sind Gegenstand der oben genannten DE-OS 2918894. Sie können nach bekannten Verfahren (vgl. z.B. DE-OS 2632603) erhalten werden, indem man z.B. bekannte Phenole der allgemeinen Formel IV

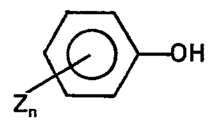

(IV)

in welcher
Z und n die oben angegebene Bedeutung haben,
mit einem Halogenketon der allgemeinen Formel V

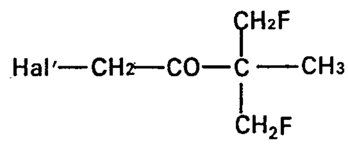

(V)

in welcher
X die oben angegebene Bedeutung hat und
Hal′ für Chlor oder Brom steht,
umsetzt. Das noch verbleibende aktive Wasserstoffatom wird anschliessend in üblicher Weise gegen Halogen ausgetauscht (vgl. auch die Herstellungsbeispiele).

Die Halogenketone der allgemeinen Formel V sind ebenfalls Gegenstand der oben genannten eigenen älteren Anmeldung. Sie können auf allgemein übliche und bekannte Weise erhalten werden, indem man Fluorderivate des 3,3-Dimethyl-butan-2-ons der allgemeinen Formel VI

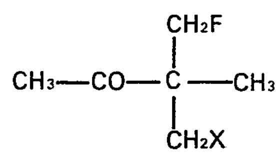

(VI)

in welcher
X die oben angegebene Bedeutung hat,
mit Chlor oder Brom in Gegenwart eines inerten organischen Lösungsmittels, wie beispielsweise Ether oder chlorierte Kohlenwasserstoffe, bei Raumtemperatur versetzt (vgl. auch die Herstellungsbeispiele), oder mit üblichen Chlorierungsmitteln, wie beispielsweise Sulfurylchlorid, bei 20 bis 60°C umsetzt.

Die Fluorderivate des 3,3-Dimethyl-butan-2-ons der allgemeinen Formel VI sind Gegenstand der DE-OS 2843767. Man erhält die Fluorderivate des 3,3-Dimethylbutan-2-ons der allgemeinen Formel VI, wenn man Sulfonsäureester der allgemeinen Formel VII

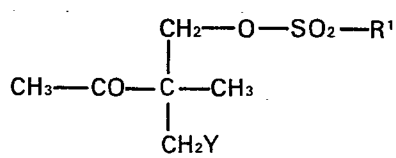

(VII)

in welcher
$R^1$ für Alkyl mit 1 bis 4 Kohlenstoffatomen, insbesondere Methyl, oder Aryl mit 6 bis 12 Kohlenstoffatomen, insbesondere Phenyl oder Tolyl, steht und
Y für Wasserstoff oder die Gruppe $-O-SO_2-R^1$ steht,
mit Metallfluoriden, wie beispielsweise Natrium- und Kaliumfluorid, in Gegenwart eines polaren organischen Lösungsmittels, wie beispielsweise Di-, Tri- oder Tetraethylenglykol, bei Temperaturen zwischen 80 und 250°C umsetzt (vgl. auch die Herstellungsbeispiele).

Die Sulfonsäureester der allgemeinen Formel VII sind teilweise bekannt [J. Org. Chem. 35, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (siehe z.B. Houben-Weyl, Methoden der Org. Chemie, Bd. IX, S. 388 und 663, sowie die Angaben bei den Herstellungsbeispielen).

Im einzelnen seien als Beispiele für die Ausgangsstoffe der allgemeinen Formel II genannt (Az steht dabei sowohl für 1,2,4-Triazol-1-yl als auch für 1,2,4-Triazol-4-yl):

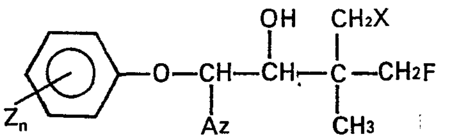

(II)

| Zn | X | Zn | X |
|---|---|---|---|
| - | H | - | F |
| 2-Cl | H | 2-Cl | F |
| 3-Cl | H | 3-Cl | F |
| 4-Cl | H | 4-Cl | F |
| 2-F | H | 2-F | F |
| 3-F | H | 3-F | F |
| 4-F | H | 4-F | F |
| 2-Br | H | 2-Br | F |
| 3-Br | H | 3-Br | F |
| 4-Br | H | 4-Br | F |
| 2,4-$Cl_2$ | H | 2,4-$Cl_2$ | F |
| 2-$CH_3$ | H | 2-$CH_3$ | F |
| 4-$CH_3$ | H | 4-$CH_3$ | F |
| 2-Cl,4-$CH_3$ | H | 2-Cl,4-$CH_3$ | F |
| 4-Cl,2-$CH_3$ | H | 4-Cl,2-$CH_3$ | F |
| 4-J | H | 4-J | F |
| 4-CN | H | 4-CN | F |
| 2-$NO_2$ | H | 2-$NO_2$ | F |
| 4-$COOCH_3$ | H | 4-$COOCH_3$ | F |
| 4-$COOC_2H_5$ | H | 4-$COOC_2H_5$ | F |
| 4- 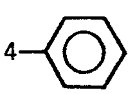 | H | 4- 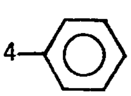 | F |
| 2- 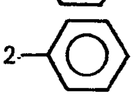 | H | 2- 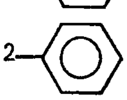 | F |
| 4- 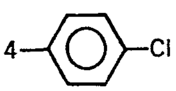 | H | 4- 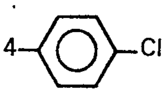 | F |

Zur Herstellung von physiologisch verträglichen Säureadditionssalzen der Verbindungen der allgemeinen Formel I kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäure, wie z.B. p-Toluolsulfonsäure und 1,5-Naphthalindisulfonsäure.

Die Säureadditions-Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren, Isolieren und gegebenenfalls durch Umkristallisation reinigen.

Die erfindungsgemässen Wirkstoffe der allgemeinen Formel I, ihre Säureadditions-Salze und Metallsalz-Komplexe weisen antimikrobielle, insbesondere starke antimykotische Wirkungen auf. Sie besitzen ein sehr breites antimykotisches Wirkungsspektrum, insbesondere gegen Dermatophyten und Sprosspilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sprosspilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Betonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummethahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Beispiel A

Antimykotische in-vitro-Wirksamkeit

Versuchsbeschreibung:

Die in-vitro-Prüfung wurde im Reihenverdünnungstest mit Keiminokula von durchschnittlich 5×10 Keimen/ml Substrat durchgeführt. Als Nährmedium dienten

a) für Dermatophyten und Schimmelpilze: Sabourand's milieu d'épresive

b) für Hefen: Isotonic Sensitest-Broth von Oxid.

Die Bebrütungstemperatur betrug 28°C; Bebrütungsdauer war 24 Stunden bei Hefen und 96 Stunden bei Dermatophyten und Schimmelpilzen.

In diesen Versuchen zeigen die erfindungsgemässen Mittel gute antimykotische Wirksamkeiten.

Beispiel B

Antimikrobielle in-vivo-Wirksamkeit (oral) bei Mäuse-Candidose

Versuchsbeschreibung:

Mäuse vom Typ SPF-$CF_1$ wurden intravenös mit $1\text{-}2\times10^6$ logaritmisch wachsenden Candida-Zellen, die in physiologischer Kochsalzlösung suspendiert waren, infiziert. Eine Stunde vor und sieben Stunden nach der Infektion wurden die Tiere mit jeweils 100 mg/kg Körpergewicht der Präparate oral behandelt.

Unbehandelte Tiere starben 3 bis 6 Tage post infectionem an der Infektion. Die Überlebensrate am 6. Tag post infectionen betrug bei unbehandelten Kontrolltieren etwa 5%.

Zeichenerklärung:

+++++ = sehr gute Wirkung = ≧90% Überlebende am 6. Tag p.i.
++++ = gute Wirkung = ≧80% Überlebende am 6. Tag p.i.
+++ = Wirkung = ≧60% Überlebende am 6. Tag p.i.
++ = schwache Wirkung = ≧40% Überlebende am 6. Tag p.i.
+ = Spur Wirkung
k.W. = keine Wirkung

Tabelle B: Antimykotische in-vivo-Wirksamkeit (oral) bei Mäuse-Candidose

| Wirkstoff | Wirkung |
|---|---|
| 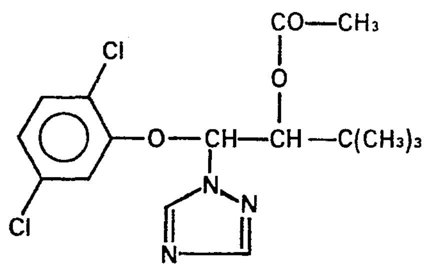 (bekannt) | k.W. |
| 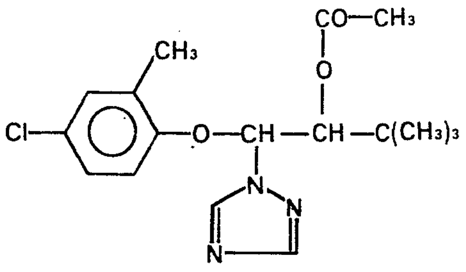 (bekannt) | k.W. |
| Verbindungen aus Bsp. Nr. | 1a +++++<br>4 +++ |

## Herstellungsbeispiele

### Beispiel 1

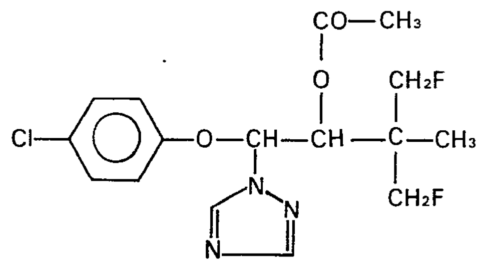

A-Form* = Bsp. 1a
B-Form* = Bsp. 1b

(Verfahren b)

112 g (0,328 Mol) 3,3-Bisfluormethyl-1-(4-chlorphenoxy)-1-(1,2,4-triazol-1-yl)-butan-2-ol (als Diastereomerengemisch der reinen Formen A und B)* werden in 500 ml Acetanhydrid gelöst. Man läst 16 Stunden bei 100°C rühren, destilliert das überschüssige Acetanhydrid im Vakuum ab und nimmt den Rückstand in 600 ml Methylenchlorid auf. Die organische Phase wird zweimal mit je 1,5 l Wasser gewaschen, über Natriumsulfat getrocknet und eingeengt. Der ölige Rückstand wird in Diisopropylether fraktioniert kristallisiert. Man erhält 48 g (38% der Theorie) 2-Acetoxy-3,3-bisfluormethyl-1-(4-chlorphenoxy)-1-(1,2,4-triazol-1-yl)-butan als A-Form vom Schmelzpunkt 130–113°C und 20,5 g (16% der Theorie) 2-Acetoxy-3,3-bisfluormethyl-1-(4-chlorphenoxy)-1-(1,2,4-triazol-1-yl)-butan als B-Form vom Schmelzpunkt 98°C.

*A- und B-Form = jeweils eine der beiden möglichen geometrischen Isomeren.

Herstellung der Vorstufen

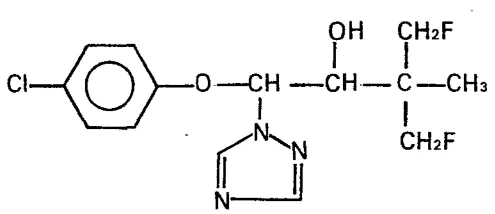

130,5 g (0,395 Mol) 3,3-Bisfluormethyl-1-(4-chlorphenoxy)-1-(1,2,4-triazol-1-yl)-2-butanon werden in 800 ml Methanol gelöst und portionsweise mit 21 g (0,55 Mol) Natriumborhydrid versetzt. Man lässt die Reaktionslösung 15 Stunden nachrühren und stellt sie dann mit konzentrierter Salzsäure auf einen pH-Wert von 3 ein. Man lässt 2 Stunden nachrühren. Nach Abdestillieren des Lösungsmittels im Vakuum wird der Rückstand mit Wasser/Natriumhydrogencarbonat versetzt und mit 600 ml Methylenchlorid ausgeschüttelt. Die vereinigten organischen Phasen werden über Natriumsulfat getrocknet und im Vakuum eingeengt. Der Rückstand kristallisiert aus Diisopropylether. Man erhält 112 g (86% der Theorie) 3,3-Bisfluormethyl-1-(4-chlorphenoxy)-1-(1,2,4-triazol-1-yl)-butan-2-ol vom Schmelzpunkt 97–102°C.

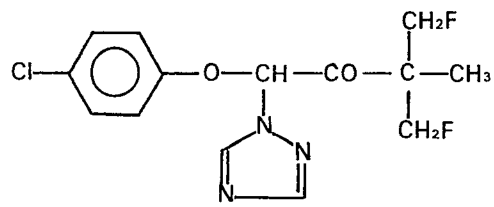

190 g (0,557 Mol) 3,3-Bisfluormethyl-1-brom-1-(4-chlorphenoxy)-butan-2-on und 90 g (1,3 Mol) Triazol werden in 800 ml Acetonitril vorgelegt, 5 Stunden bei 50°C erhitzt, das Lösungsmittel im Wasserstrahlvakuum abdestilliert, der Rückstand in einem Liter Methylenchlorid aufgenommen und zweimal mit je 1000 ml Wasser gewaschen, die organische Phase wird über Natriumsulfat getrocknet und das Lösungsmittel abdestilliert. Der Rückstand wird in 500 ml Diisopropylether aufgenommen und der Niederschlag abgesaugt. Die Mutterlauge wird destilliert. Man erhält 130,5 g (71% der Theorie) 3,3-Bisfluormethyl-1-(4-chlorphenoxy)-1-(1,2,4-triazol-1-yl)-butan-2-on vom Siedepunkt 160–66°C/0,2 mm Hg-Säule.

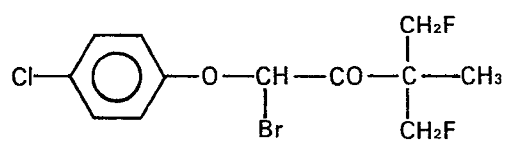

166 g (0,632 Mol) 3,3-Bisfluormethyl-1-(4-chlorphenoxy)-butan-2-on werden in 500 ml Methylenchlorid gelöst und bei 20 bis 30°C unter Rühren und Kühlen tropfenweise mit 100 g (0,625 Mol) Brom versetzt. Es wird 2 Stunden bei 20°C nachgerührt. Nach Abdestillieren des Lösungsmittels im Vakuum kristallisiert der Rückstand aus Petrolether. Man erhält 190 g (88% der Theorie) 3,3-Bisfluormethyl-1-brom-1-(4-chlorphenoxy)-butan-2-on vom Schmelzpunkt 54–57°C.

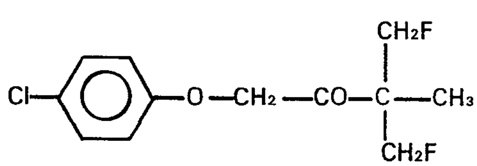

Zu einer gerührten Mischung aus 102 g (0,79 Mol) p-Chlorphenol und 110 g (0,79 Mol) gepulvertem Kaliumcarbonat in 500 ml Aceton werden bei 20 bis 30°C 171,2 g (0,79 Mol) 3,3-Bisfluormethyl-1-brom-butan-2-on zugetropft. Es wird 4 Stunden

bei 40°C nachgerührt, das anorganische Salz abfiltriert und das Filtrat eingeengt. Der Rückstand wird im Hochvakuum destilliert. Man erhält 190,5 g (90% der Theorie) 3,3-Bisfluormethyl-1-(4-chlorphenoxy)-butan-2-on vom Siedepunkt 113–117°C/0,1 mm Hg-Säule.

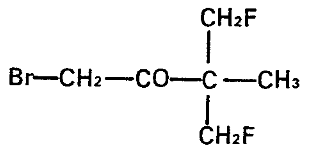

In eine Mischung aus 757,0 g (5,58 Mol) 3,3-Bisfluormethyl-2-butanon und 4,5 l Methylenchlorid werden bei 20 bis 30°C unter Kühlen und Rühren 903 g Brom langsam eingetropft. Die gelbliche Lösung wird noch 1 Stunde bei 20°C nachgerührt. Nach Abdestillieren des Lösungsmittels wird der Rückstand im Vakuum destilliert. Man erhält 1030 g (86% der Theorie) 3,3-Bisfluormethyl-1-brom-butan-2-on vom Siedepunkt 49–53°C/0,15 mm Hg-Säule.

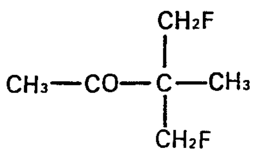

In einem Dreihalskolben mit Rührer, Tropftrichter und Liebigkühler mit gekühlter Vorlage werden 400 ml Tetraethylenglykol und 46,4 g Kaliumfluorid (0,8 Mol) vorgelegt und auf 170°C aufgeheizt. Man legt an den Vorstoss des Liebigkühlers ein Wasserstrahlvakuum (Druck ca. 20 bis 30 mbar) an. Dann werden während 45 Minuten 57,6 g (0,2 Mol) 2-Acetyl-2-methyl-propan-1,3-diol-bismethansulfat, gelöst in 10 ml Tetraethylenglykol, zugetropft. Das entstehende 3,3-Bisfluormethyl-butan-2-on wird während der Reaktion in die gekühlte Vorlage destilliert. Nach dem Zutropfen wird noch während 1 Stunde bei 175°C weiter destilliert.

Das aufgefangene Destillat wird anschliessend redestilliert. Man erhält 14 g (ca. 51,5% der Theorie) 3,3-Bisfluormethyl-butan-2-on vom Siedepunkt 43–46°C/12 mm Hg-Säule.

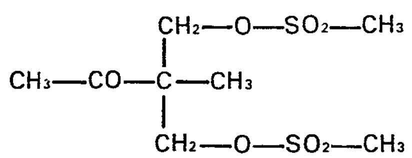

66 g (0,5 Mol) 3-Oxa-2,2-bis-(hydroxymethyl)-butan (zur Herstellung vgl. Beilstein H 1, E III 3306, IV 4132 und J. Chem. Soc., London, 1932, 2671) werden in 300 ml 1,2-Dichlorethan gelöst, 114,5 g (1 Mol) Methansulfonsäurechlorid zugetropft und bei 0 bis 5°C 158 g (2 Mol) Pyridin zugetropft. Man lässt 15 Stunden bei Raumtemperatur nachrühren und gibt dann den Ansatz auf 600 ml Eiswasser und 100 ml konzentrierter Salzsäure. Dabei fällt ein Feststoff aus, der abgesaugt wird. Die wässrige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Ether suspendiert. Der Rückstand wird abgesaugt und mit 10 ml Ether gewaschen. Man erhält 100 g (ca. 70% der Theorie) 2-Acetyl-2-methylpropan-1,3-diol-bis-methansulfonat vom Schmelzpunkt 105–108°C.

Beispiel 2

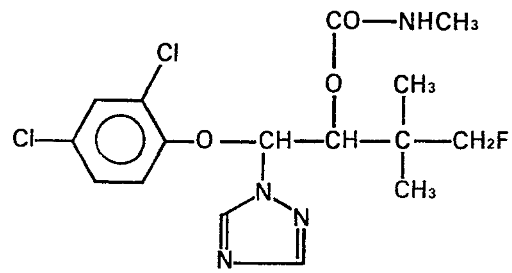

(Verfahren d)

8,4 g (0,024 Mol) 1-(2,4-Dichlorphenoxy)-3,3-dimethyl-4-fluor-1-(1,2,4-triazol-1-yl)-butan-2-ol werden in 100 ml Dioxan gelöst und mit 2 ml Methylisocyanat sowie 3,5 ml Triethylamin versetzt. Man erhitzt 10 Stunden unter Rückfluss und engt durch Abdestillieren des Lösungsmittels im Vakuum ein. Der Rückstand wird in 50 ml Essigester erhitzt und filtriert. Man erhält 8 g (86% der Theorie) 1-(2,4-Dichlorphenoxy)-3,3-dimethyl-4-fluor-2-methylcarbamoyloxy-1-(1,2,4-triazol-1-yl)-butan in Form farbloser Kristalle vom Schmelzpunkt 124–27°C.

Herstellung der Vorstufen

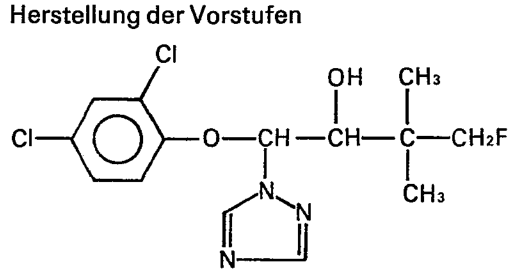

61,1 g (0,176 Mol) 1-(2,4-Dichlorphenoxy)-3,3-dimethyl-4-fluor-1-(1,2,4-triazol-1-yl)-2-butanon werden in 250 ml Methanol gelöst und portionsweise mit 3 g (0,08 Mol) Natriumborhydrid versetzt. Man lässt die Reaktionslösung 1 Stunde nachrühren und stellt sie dann mit konzentrierter Salzsäure auf einen pH-Wert von 3 ein. Nach Abdestillieren des Lösungsmittels im Vakuum wird der Rückstand mit Wasser versetzt und mit Methylenchlorid ausgeschüttelt. Die vereinigten organischen Phasen werden über Natriumsulfat getrocknet und im Vakuum eingeengt. Der Rückstand wird mit Diethylether kristallisiert. Man erhält 44,4 g (72% der Theorie) 1-(2,4-Dichlorphenoxy)-3,3-dimethyl-4-fluor-1-(1,2,4-triazol-1-yl)-2-butanol vom Schmelzpunkt 98–10°C.

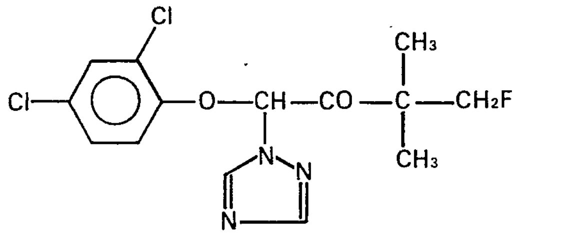

96,3 g (0,27 Mol) 1-Brom-1-(2,4-dichlorphenoxy)-3,3-dimethyl-4-fluor-2-butanon werden in 200 ml Acetonitril gelöst und zu einer siedenden Lösung von 46 g (0,56 Mol) 1,2,4-Triazol in 200 ml Acetonitril getropft. Nach 20stündigem Erhitzen unter Rückfluss wird das Lösungsmittel im Vakuum entfernt, der Rückstand mit Methylenchlorid aufgenommen, mehrmals mit Wasser gewaschen und die organische Phase über Natriumsulfat getrocknet. Nach Entfernen des Lösungsmittels im Vakuum erhält man 83 g (89% der Theorie) 1-(2,4-Dichlorphenoxy)-3,3-dimethyl-4-fluor-1-(1,2,4-triazol-1-yl)-2-butanon als Öl, das direkt weiter eingesetzt wird.

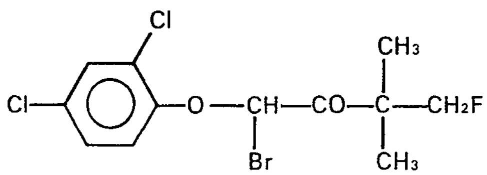

199,5 g (0,71 Mol) 1-(2,4-Dichlorphenoxy)-3,3-dimethyl-4-fluor-2-butanon werden in 500 ml Chloroform gelöst und bei 20°C unter Rühren und Kühlen tropfenweise mit 114 g (0,71 Mol) Brom versetzt. Es wird 2 Stunden bei 20°C nachgerührt, vorsichtig mit 200 ml Wasser versetzt, die Chloroformphase mehrmals mit Eiswasser gewaschen und über Natriumsulfat getrocknet. Nach Abdestillieren des Lösungsmittels im Vakuum erhält man 205,2 g (78% der Theorie) 1-Brom-1-(2,4-Dichlorphenoxy)-3,3-dimethyl-4-fluor-2-butanon als Öl, das direkt weiter umgesetzt wird.

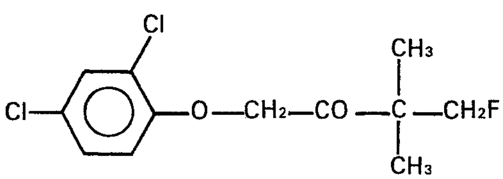

Zu einer gerührten Mischung aus 129 g (0,79 Mol) 2,4-Dichlorphenol und 110 g (0,79 Mol) gepulvertem Kaliumcarbonat in 500 ml Aceton werden unter Kühlen bei 20 bis 30°C 157 g (0,79 Mol) 1-Brom-3,3-dimethyl-4-fluor-2-butanon zugetropft. Es wird 2 Stunden bei 20°C nachgerührt, das anorganische Salz abfiltriert und das Filtrat eingeengt. Man erhält 199,3 g (90% der Theorie) 1-(2,4-Dichlorphenoxy)-3,3-dimethyl-4-fluor-2-butanon als Öl, das direkt weiter umgesetzt wird.

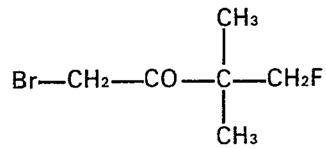

In eine Mischung aus 354 g (3 Mol) 3,3-Dimethyl-4-fluor-2-butanon und 2000 ml Ether werden bei 20 bis 30°C unter Kühlen und Rühren 480 g Brom langsam eingetropft. Die gelbliche Lösung wird noch 1 Stunde bei 20°C nachgerührt und anschliessend vorsichtig mit 500 ml Wasser versetzt. Die Etherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80–90°C/11 mm Hg-Säule.

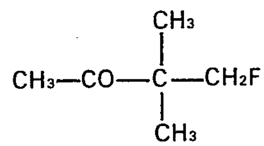

Zu der in einem Dreihals-Rührkolben mit absteigendem Kühler befindlichen Suspension von 23,2 g (0,4 Mol) trockenem Kaliumfluorid in 400 ml destilliertem Tetraethylenglykol werden bei 160°C und 20 mbar 38,8 g (0,2 Mol) 2,2-Dimethyl-2-oxobutyl-methansulfonat im Verlauf von 2 Stunden zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man erhält 20,9 g (89% der Theorie) 3,3-Dimethyl-4-fluor-2-butanon, vom Siedepunkt 130–134°C.

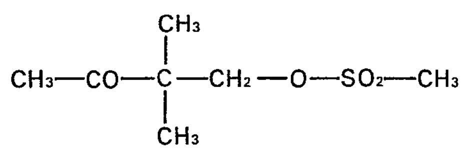

232 g (2 Mol) 3,3-Dimethyl-4-hydroxy-2-butanon (z. Herstellung vgl. Beilstein H1, E III, 3239, IV 4030 und Bull. Soc. Chim. France 1954, 2849) werden in 700 ml absolutem Pyridin bei 0 bis 5°C mit 229 g (2 Mol) Methansulfochlorid umgesetzt. Nach 12 Stunden Stehen bei 20°C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxo-butyl-methansulfonat vom Siedepunkt 106–120°C/0,12 mm Hg-Säule.

In entsprechender Weise und gemäss den Verfahren (a) bis (d) werden die nachfolgenden Beispiele der allgemeinen Formel I

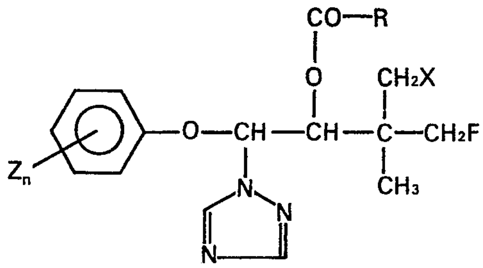

(I)

erhalten:

| Bsp. Nr. | $Z_n$ | R | X | Schmelzpunkt (°C) |
|---|---|---|---|---|
| 3 | 4-Cl | $-NHCH_3$ | H | Öl |
| 4 | 4-Cl | $-CH_3$ | H | Öl |
| 5 | 4-Cl | $-CH_2Cl$ | F | 123-25 (A-Form) |
| 6 | 4-Cl | $-CHCl_2$ | F | 214 (x1/2 NDS) |
| 7 | 4-Cl | $-NHCH_3$ | F | 144-50 (A-Form) |
| 8 | 2,4-$Cl_2$ | $-NHCH_3$ | F | 131-34 (A-Form) |
| 9 | 4-F | $-CH_3$ | F | 136-38 (A-Form) |
| 10 | 2,4-$Cl_2$ | $-CH_3$ | F | 123-25 (A-Form) |
| 11 | 4-F | $-CH_2Cl$ | F | 116-18 (A-Form) |
| 12 | 2,4-$Cl_2$ | $-CH_2Cl$ | F | 89-91 (A-Form) |
| 13 | 4-F | $-CHCl_2$ | F | 63-66 (A-Form) |
| 14 | 2,4-$Cl_2$ | $-CHCl_2$ | F | 98 (A-Form) |
| 15 | 4-F | $-NHCH_3$ | F | 181-90 (A-Form) |
| 16 | 4-F | $-NH-CH_2OCH_3$ | F | 172 (A-Form) (x 1/2 NDS) |
| 17 | 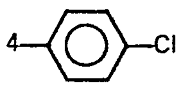 | $-CH_3$ | F | 83-87 |
| 18 | 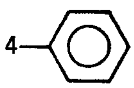 | $-NH-CH_3$ | F | 131-33 |
| 19 | 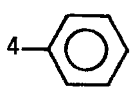 | $-NH-C_2H_5$ | F | 112-14 |
| 20 | 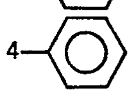 | $-NH-CH(CH_3)_2$ | F | 116-18 |
| 21 | 4-Cl | $-NH-CH(CH_3)_2$ | F | 110 |
| 22 | 2,4-$Cl_2$ | $-NH-CH(CH_3)_2$ | F | 121-22 |
| 23 | 4-Cl | 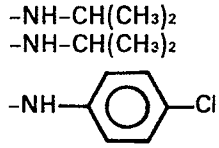 | F | 133 |
| 24 | 2,4-$Cl_2$ | 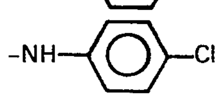 | F | 157-58 |
| 25 | 4-Cl | 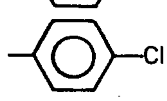 | F | 180 |
| 26 | 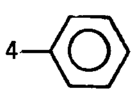 | $-NH-CH_2-O-CH_3$ | F | 180 (x1/2 NDS) |
| 27 | 4-Cl | $-NH-CH_2-O-CH_3$ | F | 180-83 (x1/2 NDS) |
| 28 | 2,4-$Cl_2$ | $-NH-CH_2-O-CH_3$ | F | 210 (x1/2 NDS) |
| 29 | 4-Cl | 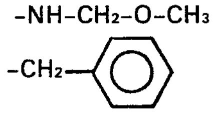 | F | 184-96 (x1/2 NDS) |
| 30 | 4-Cl | $-CH_2-O-C_2H_5$ | F | 181-88 (x1/2 NDS) |
| 31 | 4-Cl | 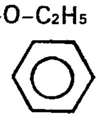 | F | 48-50 |

| Bsp. Nr. | $Z_n$ | R | X | Schmelzpunkt (°C) |
|---|---|---|---|---|
| 32 | 4-Cl | 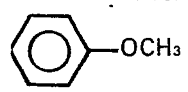 | F | 187-89 (x1/2 NDS) |
| 33 | 4-Cl | 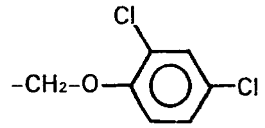 | F | 205-10 (x1/2 NDS) |
| 34 | 2,4-$Cl_2$ | 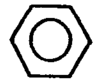 | F | 95-98 |
| 35 | 2,4-$Cl_2$ | 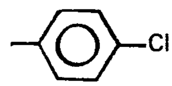 | F | 212-18 (x1/2 NDS) |
| 36 | 2,4-$Cl_2$ | $-CH_2CH_2CH_2Cl$ | F | 212-18 (x1/2 NDS) |
| 37 | 4-Cl | $-CH_2CH_2CH_2Cl$ | F | 78-81 |
| 38 | 2,4-$Cl_2$ | 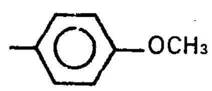 | F | 185-90 (x1/2 NDS) |
| 39 | 4-Cl | $-C(CH_3)=CH_2$ | F | 191-98 (x1/2 NDS) |
| 40 | 2,4-$Cl_2$ | 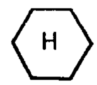 | F | 91-94 (A-Form) |
| 41 | 4-Cl | 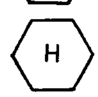 | F | 173 (A-Form) |

## Patentansprüche

1. Antimykotisches Mittel, gekennzeichnet durch einen Gehalt an mindestens einem acylierten Triazolyl-γ-fluorpinakolyl-Derivat der allgemeinen Formel I

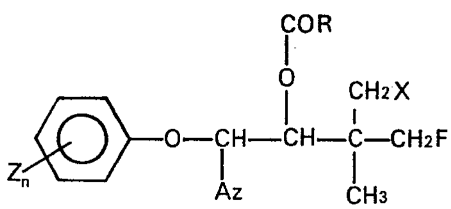

(I)

in welcher
Az für 1,2,4-Triazol-1-yl oder 1,2,4-Triazol-4-yl steht,
R für geradkettiges oder verzweigtes Alkyl mit 1-8 Kohlenstoffatomen; geradkettiges oder verzweigtes Alkenyl und Alkinyl mit jeweils 2-4 Kohlenstoffatomen; Halogenalkyl mit 1-2 Kohlenstoff- und 1-5 Halogenatomen; Alkoxy- und Alkoxyalkyl mit 1-4 Kohlenstoffatomen in jedem Alkylteil; Cycloalkyl mit 5-7 Kohlenstoffatomen; gegebenenfalls durch Halogen, Cyano, Nitro, Alkyl mit 1-2 Kohlenstoffatomen und Alkoxy mit 1-2 Kohlenstoffatomen substituiertes Phenyl, Phenylalkyl und Phenoxyalkyl mit jeweils bis zu 2 Kohlenstoffatomen im Alkylteil; Alkylamino mit 1-12 Kohlenstoffatomen; Dialkylamino mit 1-4 Kohlenstoffatomen in jedem Alkylteil; Halogenalkylamino mit bis zu 4 Kohlenstoff- und bis zu 5 gleichen oder verschiedenen Halogenatomen, Alkoxycarbonylamino und Alkoxyalkylamino mit jeweils 1-4 Kohlenstoffatomen in jedem Alkylteil; gegebenenfalls einfach oder mehrfach durch Halogen, Nitro, Cyano, geradkettiges oder verzweigtes Alkyl mit 1-4 Kohlenstoffatomen, Alkoxy und Alkylthio mit jeweils 1-2 Kohlenstoffatomen, Halogenalkyl mit bis zu 2 Kohlenstoffatomen und bis zu 5 gleichen oder verschiedenen Halogenatomen substituiertes Phenylamino; Alkoxycarbonylalkenyl mit 1-4 Kohlenstoffatomen im Alkylteil und 2-4 Kohlenstoffatomen im Alkenylteil steht;
X für Wasserstoff oder Fluor steht;
Z für Halogen, Cyano, Nitro, geradkettiges oder verzweigtes Alkyl mit bis zu 4 Kohlenstoffatomen; Cycloalkyl mit 5-7 Kohlenstoffatomen; Halogenalkyl mit bis zu 2 Kohlenstoffatomen und bis zu 5 Halogenatomen; Alkoxycarbonyl mit insgesamt bis zu 5 Kohlenstoffatomen; Alkoxy und Alkylthio mit jeweils bis zu 2 Kohlenstoffatomen; gegebenenfalls durch Halogen, Amino, Cyano, Nitro oder Alkyl mit 1-2 Kohlenstoffatomen substituiertes Phenyl und Phenoxy; gegebenenfalls im Alkylteil durch Alkylcarbonyloxy mit insgesamt 3 Kohlenstoffatomen und im Phenylteil durch Halogen, Nitro und Cyano substituiertes Phenylalkyl mit 1-2 Kohlenstoffatomen im Alkylteil steht;
n für ganze Zahlen von 0 bis 3 steht;

und deren physiologisch verträglichen Säureadditions-Salze und Metallkomplexe.

2. Antimykotisches Mittel nach Anspruch 1, gekennzeichnet durch einen Gehalt an mindestens einem acylierten Imidazolyl-γ-fluorpinakolylderivat der allgemeinen Formel I in Anspruch 1, in welcher
R für Methyl, Ethyl, Isobutyl, Chlormethyl, Dichlormethyl, Chlorethyl, Chlorpropyl, Methacryl, Cyclohexyl, gegebenenfalls einfach oder mehrfach durch Chlor, Brom, Methyl oder Methoxy substituiertes Phenyl, Benzyl oder Phenoxymethyl, für Methoxy, Ethoxy, Isopropoxy, Butoxy oder Isobutoxy, Methyl- oder Ethylamino, Dimethylamino, Phenylamino, Chlorphenylamino, Chlorethylamino, Methoxycarbonylamino, Ethoxycarbonylamino oder Methoxymethylamino steht,
X für Wasserstoff oder Fluor steht,
Z für Chlor, Brom, Methyl, Ethyl, Cyclohexyl, Methoxy, Methylthio, Trifluormethyl, Methoxycarbonyl, Cyano, Nitro oder gegebenenfalls durch Chlor substituiertes Phenyl, Benzyl oder Phenoxy steht und
n für 0, 1 oder 2 steht,
und/oder deren physiologisch verträglichen Säureadditionssalzen und Metallsalz-Komplexen.

3. Antimykotisches Mittel nach Anspruch 1, gekennzeichnet durch einen Gehalt an 2-Acetoxy-3,3-bifluormethyl-1-(4-chlorphenoxy)-1-(1,2,4-triazol-1-yl)-butan.

4. Antimykotisches Mittel nach Anspruch 1, gekennzeichnet durch einen Gehalt an 1-(2,4-Dichlorphenoxy)-3,3-dimethyl-4-fluor-1-(1,2,4-triazol-1-yl)-2-methylcarbamoyloxy-butan.

5. Antimykotisches Mittel nach Anspruch 1, gekennzeichnet durch einen Gehalt an 1-(4-Chlorphenoxy)-3,3-dimethyl-4-fluor-1-(1,2,4-triazol-1-yl)-2-methylcarbamoyloxy-butan.

6. Antimykotisches Mittel nach Anspruch 1, gekennzeichnet durch einen Gehalt an 1-(4-Chlorphenoxy)-3,3-dimethyl-4-fluor-1-(1,2,4-triazol-1-yl)-2-acetoxy-butan.

7. Verfahren zur Herstellung eines antimykotischen Mittels, dadurch gekennzeichnet, dass man mindestens ein acyliertes Triazolyl-γ-fluorpinakolylderivat gemäss der allgemeinen Formel I in Anspruch 1 mit inerten, nicht toxischen, pharmazeutisch geeigneten Trägerstoffen vermischt.

## Claims

1. Antimycotic agent, characterised in that it contains at least one acylated triazolyl-γ-fluoropinacolyl derivative of the general formula I

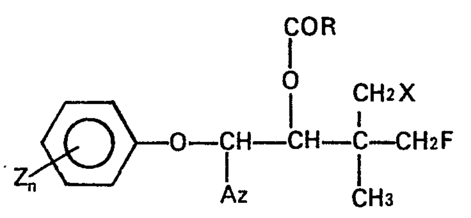

(I)

in which
Az represents 1,2,4-triazol-1-yl or 1,2,4-triazol-4-yl,
R represents straight-chain or branched alkyl with 1-8 carbon atoms; straight-chain or branched alkenyl and alkinyl with in each case 2-4 carbon atoms; halogenoalkyl with 1-2 carbon atoms and 1-5 halogen atoms; alkoxy and alkoxyalkyl with 1-4 carbon atoms in each alkyl part; cycloalkyl with 5-7 carbon atoms; phenyl, phenylalkyl and phenoxyalkyl which is optionally substituted by halogen, cyano, nitro, alkyl with 1-2 carbon atoms and alkoxy with 1-2 carbon atoms and in each case has up to 2 carbon atoms in the alkyl part; alkylamino with 1-12 carbon atoms; dialkylamino with 1-4 carbon atoms in each alkyl part; halogenoalkylamino with up to 4 carbon atoms and up to 5 identical or different halogen atoms, alkoxycarbonylamino and alkoxyalkylamino with in each case 1-4 carbon atoms in each alkyl part; phenylamino which is optionally monosubstituted or polysubstituted by halogen, nitro, cyano, straight-chain or branched alkyl with 1-4 carbon atoms, alkoxy and alkylthio with in each case 1-2 carbon atoms, halogenoalkyl with up to 2 carbon atoms and up to 5 identical or different halogen atoms; alkoxycarbonylalkenyl with 1-4 carbon atoms in the alkyl part and 2-4 carbon atoms in the alkenyl part;
X represents hydrogen or fluorine;
Z represents halogen, cyano, nitro, straight-chain or branched alkyl with up to 4 carbon atoms; cycloalkyl with 5-7 carbon atoms; halogenoalkyl with up to 2 carbon atoms and up to 5 halogen atoms; alkoxycarbonyl with a total of up to 5 carbon atoms; alkoxy and alkylthio with in each case up to 2 carbon atoms; phenyl and phenoxy optionally substituted by halogen; amino, cyano, nitro or alkyl with 1-2 carbon atoms; phenylalkyl which has 1-2 carbon atoms in the alkyl part and is optionally substituted in the alkyl part by alkylcarbonyloxy with a total of 3 carbon atoms and in the phenyl part by halogen, nitro and cyano;
n represents integers from 0 to 3;
and physiologically acceptable acid addition salts and metal complexes thereof.

2. Antimycotic agent according to Claim 1, characterised in that it contains at least one acylated triazolyl-γ-fluoropinacolyl derivative of the general formula I in Claim 1, in which
R represents methyl, ethyl, isobutyl, chloromethyl, dichloromethyl, chloroethyl, chloropropyl, methacryl or cyclohexyl, phenyl, benzyl or phenoxymethyl which is optionally monosubstituted or polysubstituted by chlorine, bromine, methyl or methoxy, or methoxy, ethoxy, isopropoxy, butoxy or isobutoxy, methyl- or ethyl-amino, dimethylamino, phenylamino, chlorophenylamino, chloroethylamino, methoxycarbonylamino, ethoxycarbonylamino or methoxymethylamino,
X represents hydrogen or fluorine,
Z represents chlorine, bromine, methyl, ethyl, cyclohexyl, methoxy, methylthio, trifluoromethyl, methoxycarbonyl, cyano, nitro or phenyl, benzyl or phenoxy which is optionally substituted by

chlorine and
n represents 0, 1 or 2,
and/or physiologicaly acceptable acid addition salts and metal salt complexes thereof.

3. Antimycotic agent according to Claim 1, characterised in that it contains 2-acetoxy-3,3-bifluoromethyl-1-(4-chlorophenoxy)-1-(1,2,4-triazol-1-yl)-butane.

4. Antimycotic agent according to Claim 1, characterised in that it contains 1-(2,4-dichlorophenoxy)-3,3-dimethyl-4-fluoro-1-(1,2,4-triazol-1-yl)-2-methylcarbamoyloxy-butane.

5. Antimycotic agent according to Claim 1, characterised in that it contains 1-(4-chlorophenoxy)-3,3-dimethyl-4-fluoro-1-(1,2,4-triazol-1-yl)-2-methylcarbamoyloxy-butane.

6. Antimycotic agent according to Claim 1, characterised in that it contains 1-(4-chlorophenoxy)-3,3-dimethyl-4-fluoro-1-(1,2,4-triazol-1-yl)-2-acetoxy-butane.

7. Process for the preparation of an antimycotic agent, characterised in that at least one acylated triazolyl-γ-fluoropinacolyl derivative according to the general formula I in Claim 1 is mixed with inert, non-toxic, pharmaceutically suitable excipients.

**Revendications**

1. Agent antimycotique, caractérisé en ce qu'il contient au moins un dérivé acylé de triazolyl-γ-fluoropinacolyle de formule générale I

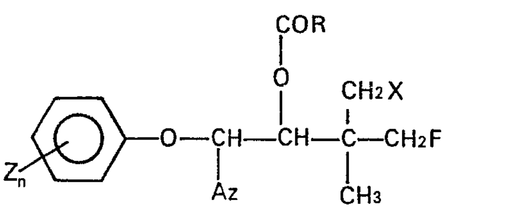

(I)

dans laquelle
Az représente le groupe 1,2,4-triazole-1-yle ou le groupe 1,2,4-triazole-4-yle,
R représente un groupe alkyle à chaîne droite ou ramifiée contenant de 1 à 8 atomes de carbone; un groupe alcényle et alcynyle à chaîne droite ou ramifiée contenant chacun 2 à 4 atomes de carbone; halogénoalkyle contenant 1 à 2 atomes de carbone et 1 à 5 atomes d'halogène; alcoxy et alcoxyalkyle contenant 1 à 4 atomes de carbone dans chacune des parties alkyle; cycloalkyle contenant 5 à 7 atomes de carbone; phényle, phénylalkyle, phénoxyalkyle contenant chacun jusqu'à 2 atomes de carbone dans la partie alkyle, éventuellement substitué par des halogènes, des groupes cyano, nitro, alkyle en $C_1$–$C_2$ et alcoxy en $C_1$–$C_2$; alkylamino en $C_1$–$C_{12}$; dialkylamino contenant 1 à 4 atomes de carbone dans chacune des parties alkyle; halogénoalkylamino contenant jusqu'à 4 atomes de carbone et jusqu'à 5 atomes d'halogènes identiques ou différents; alcoxycarbonylamino et alcoxyalkylamino contenant chacun 1 à 4 atomes de carbone dans chacune des parties alkyle; phénylamino éventuellement mono- ou poly-substitué par des halogènes, des groupes nitro, cyano, alkyle à chaîne droite ou ramifiée en $C_1$–$C_4$, alcoxy et alkylthio contenant chacun 1 à 2 atomes de carbone, halogénoalkyle contenant jusqu'à 2 atomes de carbone et jusqu'à 5 atomes d'halogènes identiques ou différents; alcoxycarbonylalcényle contenant 1 à 4 atomes de carbone dans la partie alkyle et 2 à 4 atomes de carbone dans la partie alcényle;
X représente l'hydrogène ou le fluor;
Z représente un halogène, un groupe cyano, nitro, alkyle à chaîne droite ou ramifiée contenant jusqu'à 4 atomes de carbone; cycloalkyle contenant 5 à 7 atomes de carbone; halogénoalkyle contenant jusqu'à 2 atomes de carbone et jusqu'à 5 atomes d'halogènes; alcoxycarbonyle contenant au total jusqu'à 5 atomes de carbone; alcoxy et alkylthio contenant chacun jusqu'à 2 atomes de carbone; phényle et phénoxy éventuellement substitué par des halogènes, des groupes amino, cyano, nitro ou alkyle en $C_1$–$C_2$; phénylalkyle contenant 1 à 2 atomes de carbone dans la partie alkyle et éventuellement substitué dans la partie alkyle par un groupe alkylcarbonyloxy contenant au total 3 atomes de carbone et, dans la partie phényle par des halogènes, des groupes nitro et cyano;
n représente des nombres entiers de 0 à 3;
et leurs sels formés par addition avec des acides et complexes métalliques tolérés par l'organisme.

2. Agent antimycotique selon la revendication 1, caractérisé en ce qu'il contient au moins un dérivé acylé de triazolyl-γ-fluoropinacolyle de formule générale I de la revendication 1, dans laquelle:
R représente un groupe méthyle, éthyle, isobutyle, chlorométhyle, dichlorométhyle, chloréthyle, chloropropyle, méthacryle, cyclohexyle, phényle, benzyle ou phénoxyméthyle éventuellement mono- ou polysubstititué par le chlore, le brome, des groupes méthyle ou méthoxy, un groupe méthoxy, éthoxy, isopropoxy, butoxy ou isobutoxy, méthyl- ou éthylamino, diméthylamino, phénylamino, chlorophénylamino, chloréthylamino, méthoxycarbonylamino, éthoxycarbonylamino ou méthoxyméthylamino;
X représente l'hydrogène ou le fluor;
Z représente le chlore, le brome, un groupe méthyle, éthyle, cyclohexyle, méthoxy, méthylthio, trifluorométhyle, méthoxycarbonyle, cyano, nitro ou phényle, benzyle ou phénoxy éventuellement substitué par le chlore, et
n est égal à 0, 1 ou 2,
et/ou leurs sels formés par addition avec des acides et complexes de sels métalliques tolérés par l'organisme.

3. Agent antimycotique selon la revendication 1, caractérisé en ce qu'il contient du 2-acétoxy-3,3-bifluorométhyl-1-(4-chlorophénoxy)-1-(1,2,4-triazole-1-yl)-butane.

4. Agent antimycotique selon la revendication 1, caractérisé en ce qu'il contient du 1-(2,4-dichlorophénoxy)-3,3-diméthyl-4-fluoro-1-(1,2,4-triazole-1-yl)-2-méthylcarbamoyloxy-butane.

5. Agent antimycotique selon la revendication 1, caractérisé en ce qu'il contient du 1-(4-chlorophénoxy)-3,3-diméthyl-4-fluoro-1-(1,2,4-triazole-1-yl)-2-méthylcarbamoyloxy-butane.

6. Agent antimycotique selon la revendication 1, caractérisé en ce qu'il contient du 1-(4-chlorophénoxy)-3,3-diméthyl-4-fluoro-1-(1,2,4-triazole-1-yl)-2-acétoxy-butane.

7. Procédé de préparation d'un agent antimycotique, caractérisé en ce que l'on mélange au moins un dérivé acylé de triazolyl-γ-fluoropinacolyle de formule générale I de la revendication 1 avec des véhicules inertes non toxiques appropriés à l'usage pharmaceutique.